# EUROPÄISCHE PATENTSCHRIFT

(11) **EP 0 781 998 B1**
(45) Veröffentlichungstag und Bekanntmachung des Hinweises auf die Patenterteilung: **10.12.2003**
(21) Anmeldenummer: 96118937.0
(22) Anmeldetag: 27.11.1996
(51) Int. Cl.: G01N 33/543, G01N 33/563, G01N 33/576, G01N 33/58

(54) **Immunologische Bestimmungsmethode**
Immunological determination method
Méthode de détermination immunologique

(30) Priorität: 27.12.1995 DE 19548375
(43) Veröffentlichungstag der Anmeldung: 02.07.1997
(73) Patentinhaber: Dade Behring Marburg GmbH, 35001 Marburg (DE)
(72) Erfinder: Pauly, Hans-Erwin, Dr., 35232 Dautphetal (DE); Peiseler-Müller, Hanna, 35043 Marburg (DE)

(56) Entgegenhaltungen:
- EP-A- 0 363 942
- DE-A- 3 202 559

## Beschreibung

Die vorliegende Erfindung betrifft eine immunchemische Bestimmungsmethode nach dem kompetitiven Prinzip, bei der die Konzentration des Analyten in einem 1-Schritt-Verfahren bestimmt wird und der Analyt dabei mit einem ersten und einem zweiten spezifischen Bindungspartner um die Bindung an einen dritten spezifischen Bindungspartner konkurriert, wobei der erste spezifische Bindungspartner an einen wasserunlöslichen Träger gebunden und der zweite spezifische Bindungspartner mit einer signalerzeugenden Markierung versehen ist.

Immunologische Bestimmungsmethoden haben seit der ersten Beschreibung eines Radioimmunoassays (1959) und des ersten Enzymimmunoverfahrens in vielen Bereichen der klinischen Diagnostik eine überragende Bedeutung gewonnen.

Bei einem Enzymimmunoverfahren zur Bestimmung eines Analyten kommen immunologische Bindungs- und Reaktionspartner zur Anwendung, wie z. B. Haptene, Antigene, Antikörper oder Fragmente von Antikörpern. Die verwendeten Bindungs- und Reaktionspartner können dabei zum einen an einer Festphase gebunden vorliegen oder mit einem signalerzeugenden Label, z. B. einem Markierungsenzym über z. B. eine kovalente Bindung konjugiert sein. Als Festphase werden konkave Formkörper verwendet, wie z. B. Röhrchen oder Vertiefungen in Form von Mikrotitrationsplatten, aber auch konvexe Formkörper, wie z. B. Kugeln. Planare Festphasen, wie z. B. Teststreifen, finden ebenfalls Anwendung. Als Markierungsenzyme werden häufig alkalische Phosphatase, β-Galaktosidase und Meerrettich-Peroxidase eingesetzt, wobei als Substrate chromogene, fluorogene oder lumineszente Verbindungen verwendet werden. Die Zusammensetzung von Proben-, Inkubations- und Waschpuffern, sowie die verschiedenen Substrat-/Chromogen-Reagenzien sind dem Fachmann bekannt.

Im Unterschied zu homogenen Enzymimmunoverfahren werden bei heterogenen Enzymimmunoverfahren ungebundene Reaktionspartner von gebundenen Reaktionspartnern durch eine Phasentrennung und anschließende Waschschritte entfernt. Dabei wird zwischen dem 1-Schritt- und dem 2-Schritt-Verfahren unterschieden. Während bei dem 1-Schritt-Verfahren insgesamt nur ein Separationsschritt ("bound/free"-Trennung) durchgeführt wird, erfolgt dies bei dem 2-Schritt-Verfahren nach jedem einzelnen Inkubations- bzw. Reaktionsschritt.

Generell werden Enzymimmunoverfahren nach den immunologischen Reaktionsprinzipien in nicht-kompetitive und kompetitive Techniken eingeteilt. Die nicht-kompetitiven Techniken ("Sandwich"-Teste) zeichnen sich dadurch aus, daß festphasengebundene und signalerzeugende Reaktionspartner im Vergleich zu dem zu bestimmenden Analyten im großen molaren Überschuß vorliegen. Für die Ausbildung des "Sandwich"-Komplexes sind mindestens zwei Bindungsstellen des Analyten notwendig, die jeweils von den festphasengebundenen bzw. signalerzeugenden Reaktionspartnern erkannt werden. Die gemessene Signalaktivität des gebildeten "Sandwich"-Komplexes ist dabei direkt proportional zur Analytkonzentration.

Bei den kompetitiven Techniken ist dagegen einer der Reaktionspartner durch seine geringe Konzentration für die Immunkomplexbildung limitierend, so daß eine Konkurrenz zwischen Reaktionspartner und Analyt um mindestens eine Bindungsstelle des gemeinsamen Bindungspartners stattfindet. Innerhalb der kompetitiven Techniken lassen sich diese ihrerseits in zwei Gruppen unterteilen. Bei der ersten Gruppe ist die Anzahl der insolubilisierten Bindungspartner geringer als die Anzahl der signalerzeugenden Reaktionspartner und der zu bestimmenden Analytmoleküle, während bei der zweiten Gruppe die Konzentration des signalerzeugenden Reaktionspartners geringer ist als die Anzahl der insolubilisierten Bindungspartner und freier Analytmoleküle. In beiden Fällen ist die Signalaktivität des gebildeten Komplexes umgekehrt proportional zu der gemessenen Analytkonzentration.

Bei einem Vergleich der beiden immunologischen Reaktionsprinzipien kann festgestellt werden, daß die bekannten kompetitiven Techniken nicht-kompetitiven Techniken in Bezug auf Sensitivität, Meßbereich, Spezifität, Robustheit und Inkubationszeit unterlegen sind (EKINS R. (1985) CURRENT CONCEPTS AND FUTURE DEVELOPMENTS: IN ALTERNATIVE IMMUNOASSAYS).

Verschiedene Ausführungsformen von Immunoassays sind im Stand der Technik bereits bekannt. In der EP-A-0 363 942 beispielsweise wird ein kompetitiver Immunoassay beschrieben, der auf der Kompetition von drei Bindungspartnem um einen Analyten beruht, wobei es sich bei dem nachzuweisenden Analyten um ein Antigen handelt, das selbst kein Antikörper ist. Ein auf dem sogenannten Anti-µ-Prinzip beruhender Immunoassay zum Nachweis von Antikörpern gegen das Hepatitis A-Virus wird in der DE 32 02 559 A beschrieben.

Die unterschiedliche Affinität der nachzuweisenden Serumantikörper bestimmt daher ganz wesentlich, ob das 1-Schritt- oder 2-Schritt-Verfahren eingesetzt wird. Beim Nachweis niederaffiner Antikörper hat sich das 2-Schritt-Verfahren gegenüber dem 1-Schritt-Verfahren als vorteilhaft erwiesen, insbesondere wenn man die Seruminkubationszeit als ersten Schritt über Nacht durchführt. Es ist aber darauf zu achten, daß sich im zweiten Inkubationsschritt kein Gleichgewicht zwischen den gebundenen und ungebundenen signalerzeugenden Reaktionspartnern einstellt, was zu einer Verdrängung niederaffiner Analytantikörper führen würde. Der Nachweis niederaffiner Antikörper ist somit mit dem 2-Schritt-Verfahren prinzipiell besser machbar als mit dem für den Anwender leichter durchzuführenden und kürzeren 1-Schritt-Verfahren.

Die Reinheit des festphasengebundenen Antigens, um dessen Bindung in einem kompetitiven Enzymimmunoverfahren sowohl die nachzuweisenden Antikörper als auch die signalerzeugenden Reaktionspartner konkurrieren, spielt eine wichtige Rolle. Entscheidend für die Empfindlichkeit des Nachweises ist dabei die Epitopdichte des insolubilisierten Antigens (KENNY G. et al. (1983) J. CLIN. MICROBIOL. 17, 655-665). Wird ein Proteingemisch eingesetzt, wie es z. B. bei einem aufgereinigtem HAV-Virusantigen der Fall ist, so kann die Empfindlichkeit des Nachweises dann erheblich beeinträchtigt werden, wenn die virusspezifischen Proteinanteile nur einen geringen Anteil am eingesetzten Gesamtprotein darstellen. Um dies zu vermeiden, muß daher bei den bekannten Verfahren ein entsprechend hochgereinigtes Antigen verwendet werden, wobei die Herstellung des erforderlichen Reinheitsgrades mit einem erheblichen Aufwand verbunden ist und sich entsprechend schwierig, teuer und zeitaufwendig gestaltet (PURCELL R. et al. (1976) JOURNAL OF IMMUNOLOGY 116, 349-356). Insbesondere bei dem Nachweis von niederaffinen Anti-HAV-Antikörpem, die unmittelbar nach erfolgter Impfung gebildet werden, zeigen die verschiedenen kommerziell erhältlichen Diagnostica-Tests eine schlechte Sensitivität. Erst durch den Einsatz von gereinigtem HAV-Antigen bzw. dem Impfstoff-Antigen konnte die Sensitivität beim Nachweis von niederaffinen Antikörpern verbessert werden (DELEM A. (1992) BIOLOGICALS 20, 289-291).

Die der vorliegenden Erfindung zugrunde liegende Aufgabe bestand daher darin, ein Verfahren zu entwickeln, das den Nachweis von niederaffinen Antikörpern mit Hilfe des 1-Schritt-Verfahrens erlaubt und dabei ein nur minimal aufgereinigtes Antigen verwendet werden kann.

Gelöst wurde diese Aufgabe im wesentlichen durch die in den Patentansprüchen bereitgestellten Ausführungsformen.

Das erfindungsgemäße immunchemische Verfahren zur Bestimmung eines Analyten mittels eines heterogenen kompetitiven Bestimmungsverfahrens, schließt folgende Schritte ein:
a) Inkubation des Analyten mit einem ersten, einem zweiten und einem dritten spezifischen Bindungspartner, wobei der erste spezifische Bindungspartner an eine wasserunlösliche Festphase gebunden ist (Festphasengebundener Reaktionspartner) und der zweite spezifische Bindungspartner mit einer signalerzeugenden Markierung versehen ist (signalerzeugender Reaktionspartner), und der Analyt und der erste und der zweite spezifische Bindungspartner um die Bindung an den dritten spezifischen Bindungspartner (gemeinsamer Bindungspartner) konkurrieren,
b) Trennung der über den dritten spezifischen Bindungspartner an die Festphase gebundenen signalerzeugenden Markierung von dem ungebundenen Anteil,
c) Messung des durch den gebundenen Anteil der Markierung generierbaren Signals, und
d) Bestimmung der Analytkonzentration durch Vergleich der in Schritt c) gefundenen Werte mit einer unter gleichen Bedingungen aufgestellten oder theoretisch berechneten Standardkurve,
dadurch gekennzeichnet, daß der Analyt ein Hepatitis A-Virus- (HAV-) spezifischer Antikörper ist, daß als erster spezifischer Bindungspartner ein polyklonaler gegen HAV gerichteter Antikörper oder entsprechende Antikörperfragmente, als zweiter spezifischer Bindungspartner ein monoklonaler gegen HAV gerichteter Antikörper oder entsprechende Antikörperfragmente und als dritter spezifischer Bindungspartner ein HAV-Antigen verwendet wird, und daß der erste und zweite spezifische Bindungspartner unterschiedliche Bindungsstellen auf dem dritten Bindungspartner erkennen.

Festphasen als solche sind dem Fachmann an sich bekannt. Vorteilhafterweise werden wasserunlösliche Festphasen eingesetzt, wie z. B. Latexpartikel, magnetisch anziehbare Partikel oder Mikrotitrationsplatten.

Signalerzeugende Markierungen als solche sind dem Fachmann an sich bekannt. Eine solche Markierung kann entweder direkt an den betreffenden spezifischen Bindungspartner konjugiert sein oder über eine dem Fachmann an sich bekannte reversible Kopplung wie z. B. Biotin-Streptavidin, fos-jun oder Antikörper-Antigen Bindungen.

Als signalerzeugende Markierung werden bevorzugterweise Komponenten, die zur Chemolumineszenz oder Fluoreszenz befähigt sind eingesetzt oder Enzyme die luminogene, fluorogene oder chromogene Substrate umsetzen können. Bei den Enzymen besonders bevorzugt ist die Meerrettichperoxidase. Bei den chemolumineszenten Markierungen sind besonders bevorzugt die in den europäischen Patentanmeldungen EP-A-0 257 541 und EP-A-0 330 050 beschriebenen Verbindungen.

Üblicherweise wird bei den beschriebenen immunchemischen Verfahren nach der Trennung der festen und der flüssigen Phase das Signal entweder an der festen Phase oder in dem abgetrennten Überstand gemessen. Aus dem gemessenen Signal wird in an sich dem Fachmann bekannter Weise mittels einer sogenannten Standardkurve die Analytkonzentration bestimmt. Zur Erstellung der Standardkurve werden unter Verwendung des jeweiligen Bestimmungsverfahrens für bekannte Analytkonzentrationen die Signale gemessen und entweder graphisch oder mathematisch in eine Kurvenform umgesetzt. Eine solche Standardkurve kann auch mit einer gewissen Genauigkeit aufgrund der bekannten physikalischen und chemischen Eigenschaften der spezifischen Bindungspartner theoretisch berechnet werden.

Das erfindungsgemäße Verfahren ist anwendbar bei immunchemischen Methoden, bei denen eine Kompetition zwischen dem zu bestimmenden Analyten und den signalerzeugenden Reaktionspartnern und/oder insolubilisierten Reaktionspartnern um die Bindung an einen gemeinsamen Bindungspartner stattfindet.

Das erfindungsgemäße Verfahren zeichnet sich weiterhin dadurch aus, daß der gemeinsame Bindungspartner nur minimal aufgereinigt werden muß. Es hat sich als wesentlich herausgestellt, daß der gemeinsame Bindungspartner mindestens zwei unterschiedliche Bindungsstellen aufweisen sollte, wobei eine Bindungsstelle von den insolubilisierten Reaktionspartnern erkannt wird, während die signalerzeugenden Reaktionspartner an die zweite Bindungsstelle binden. Bei Vorhandensein des nachzuweisenden Analyten erfolgt dann eine spezifische Kompetition der Analytmoleküle mit den signalerzeugenden und/oder den insolubilisierten Reaktionspartnern um die Bindung an den gemeinsamen Bindungspartner. Als wesentlich für das erfindungsgemäße Ver-fahren hat sich dabei ergeben, daß bei Nichtvorhandensein eines Analyten die Ausbildung eines signalbildenden "Sandwich"-Komplexes nicht durch die Kompetition zwischen insolubilisierten und signalerzeugenden Reaktionspartnem verhindert wird.

Überraschenderweise wurde beim erfindungsgemäßen Verfahren zum Nachweis von Antikörpern gegen das Hepatitis-A-Virus festgestellt, daß bei Nichtvorhandensein eines Analyten (= negative Kontrolle) keine Kompetition zwischen den signalerzeugenden Reaktionspartnern (= monoklonales Anti-HAV-spezifisches Antikörperkonjugat) und den insolubilisierten Reaktionspartnern (= polyklonale Anti-HAV-spezifische Antikörper) um die Bindung an den gemeinsamen Bindungspartner (= HAV-Antigen) auftrat und dadurch die Ausbildung eines signalerzeugenden "Sandwich"-Komplexes ermöglicht wird. Dies war insofern überraschend, weil die bekannten monoklonalen Antikörper gegen HAV die Bindung von polyklonalen Antikörpern an das Virus in verschiedenen kompetitiven Immunoverfahren fast vollständig blockieren können (LEMON S. et al. (1993) VIROLOGY 4, 285-295; HUGHES J. et al. (1984) J. VIROL. 52, 465-473; STAPLETON J. et al. (1987) J. VIROL. 61, 491-498). Dagegen konnte bei Vorhandensein von Analytmolekülen (= Anti-HAV-spezifische Antikörper = positive Kontrolle) ebenso überraschend festgestellt werden, daß eine spezifische Kompetition um den gemeinsamen Bindungspartner zwischen dem Analyten und den insolubilisierten Reaktionspartnern und/oder signalerzeugenden Reaktionspartnern stattfinden kann.

Überraschend war auch die Feststellung, daß der gemeinsame Bindungspartner nur minimal oder gar nicht aufgereinigt werden mußte, um bereits mit dem 1-Schritt-Verfahren niederaffine Antikörper nachzuweisen, die in der Frühphase einer Infektion oder nach erfolgter Impfung auftreten können. Im erfindungsgemäßen Verfahren werden möglicherweise durch das Vorhandensein von festphasengebundenen Reaktionspartnern Voraussetzungen geschaffen, die eine besonders effektive und spezifische Kompetition zwischen dem Analyten und den signalerzeugenden und/oder festphasengebundenen Reaktionspartnern um die Bindungsstellen des gemeinsamen Bindungspartners erlauben. Minimal aufgereinigt im Sinne der vorliegenden Erfindung heißt dabei, daß der Anteil an spezifischem Protein kleiner als 80, bevorzugterweise kleiner als 50 %, ganz bevorzugterweise kleiner als 20 % ist.

Vorzugsweise werden im Rahmen der Erfindung als Reaktionspartner Antikörper oder definierte Fragmente von Antikörpern verwendet. Die Herstellung von polyklonalen bzw. monoklonalen Antikörpern (KÖHLER G. und MILSTEIN C. (1975) NATURE 256, 495-497) erfolgt nach einer dem Fachmann an sich bekannten Methode. Neben polyklonalen Antikörpern können auch monoklonale Antikörper oder deren Fragmente (F(ab')₂ oder Fab') eingesetzt werden. Entsprechend dem erfindungsgemäßen Verfahren wird dabei ein Reaktionspartner an die wasserunlösliche Festphase gebunden, während der zweite Reaktionspartner als signalerzeugende Komponente eingesetzt wird. Gemäß dem erfindungsgemäßen Verfahren wird ein polyklonaler Antikörper an die Festphase gebunden und ein monoklonaler Antikörper, der mit einem Markierungsenzym konjugiert ist, als signalerzeugender Reaktionspartner verwendet. Die Präparation des in der Erfindung verwendeten monoklonalen Konjugates ist dem Fachmann ebenfalls bekannt (Übersichtsartikel: ISHIKAWA E. et al. (1983) J. IMMUNOASSAY 4, 209-327).

Die Eignung der verwendeten Antikörper kann z. B. durch dem Fachmann an sich bekannte Experimente bestimmt werden.

Das erfindungsgemäße Verfahren läßt sich auf alle immunologischen Nachweismethoden anwenden, bei denen in heterologen Immunoassays eine spezifische Kompetition zwischen dem Analyten und festphasengebundenem Reaktionspartner und/oder signalerzeugenden Reaktionspartner um mindestens zwei Bindungsstellen eines gemeinsamen Bindungspartners stattfinden kann.

Die folgenden Beispiele sollen die Erfindung erläutern:

### Abkürzungen:

- HAV:: Hepatitis-A-Virus
- POD:: Peroxidase
- SH:: Sulfhydrylgruppen
- ATCC:: American Tissue Cell Culture

### Beispiele

### Beispiel 1

### a): Konjugation von Antikörpern

Monoklonale Antikörper gegen HAV werden mit einem heterobifunktionellen Reagenz umgesetzt (TARRIMORE et al. (1983) J. IMM. METH. 62, 123-131), danach mit SH-aktivierter Peroxidase (KING et al. (1978) BIOCHEMISTRY 17, 1499-1506) inkubiert und anschließend gelchromatographisch gereinigt.

### b): Präparation von HAV-Antigen

Zur Herstellung des HAV-Antigens werden kommerziell erhältliche Zellen, wie z. B. humandiploide embryonale Lungenfibroblasten mit einem charakterisierten Hepatitis-A-Virusstamm, wie z. B. ATCC HM-175, infiziert. Nach mehreren Tagen wird der abgenommene Zellüberstand zentrifugiert, das erhaltene Zellpellet in einem üblichen Lagerpuffer aufgenommen und das Antigen nach einem dem Fachmann bekannten Verfahren inaktiviert. Das inaktivierte Antigen kann ohne zusätzliche Aufreinigung weiter verwendet werden.

Das erfindungsgemäße Verfahren beschränkt sich dabei nicht auf die o. g. Methode zur Präparation von HAV-Antigen, sondern auch andere, dem Fachmann bekannte Präparationsverfahren zur Antigenisolierung können verwendet werden. Darüber hinaus können auch kommerziell-erhältliche HAV-Antigenpräparationen in dem erfindungsgemäßen Verfahren eingesetzt werden.

### c): Beschichtung der Vertiefungen von Mikrotitrationsplatten

Zu einer Beschichtungslösung (Natriumcarbonat 0,01 mol/l pH 9,6) wird unter leichtem Rühren eine bestimmte Menge von humanen polyklonalen Anti-HAV-Antikörper (16 µg/ml) zugegeben und ca. 30 Minuten homogenisiert. Die Beschichtung der einzelnen Vertiefungen einer Mikrotitrationsplatte erfolgte anschließend mit einem Beschichtungsvolumen von 150 µl. Nach einer Inkubation über Nacht bei Raumtemperatur wird die Beschichtungslösung abgesaugt und die einzelnen Vertiefungen der Mikrotitrationsplatte werden mit einer Waschlösung (0,25 mol/l Zitronensäure / 0,05 mol/l Tris pH 7,4) zweimal gewaschen. Im Anschluß an den letzten Waschschritt werden die einzelnen Vertiefungen der Mikrotitrationsplatte leergesaugt und zusammen mit verpacktem Trockenmittel (z. B. Silikagel) in Alufolien eingeschweißt. Die beschichteten Miktotitrationsplatten werden bei 4°C bis zu ihrer Verwendung gelagert.

### d): Enzymimmunoverfahren zur Bestimmung von Anti-HAV-Antikörpern

Das erfindungsgemäße Verfahren ist ein kompetitiver Enzymimmunotest nach dem 1-Schritt-Verfahren. In den Vertiefungen einer Mikrotitrationsplatte, die mit humanen polyklonalen Anti-HAV-spezifischen Antikörpern beschichtet sind, werden nacheinander Untersuchungsprobe (25 µl), Konjugat (=POD-konjugierter Anti-HAV-spezifischer monoklonaler Antikörper, 50 µl) und HAV-Antigen (50 µl) zugegeben. Die beschichtetete Mikrotitrationsplatte, Konjugat und HAV Antigen wurden dem Enzygnost® anti HAV Testkit (Best. Nr. OQEC Behringwerke AG, Marburg) entnommen. Enthält dabei die Untersuchungsprobe die zu bestimmenden Anti-HAV-Antikörper, so konkurrieren diese mit den Konjugatmolekülen und/ oder insolubilisierten polyklonalen Anti-HAV-spezifischen Antikörpern um die Bindung an das HAV-Antigen. Nach einer Inkubationszeit von 2 Stunden bei 37 °C werden überschüssiges Konjugat und ungebundenen Reaktanden durch Absaugen und viermaliges Waschen, z. B. mit dem Behring ELISA Processor II oder Behring ELISA Processor III (Behringwerke AG, Marburg), entfernt und die Menge des gebundenen Konjugates durch die Zugabe von 100 µl Substrat/Chromogen-Lösung (Behringwerke AG, Best. Nr. OUVP) bestimmt (30 min, Raumtemperatur, lichtgeschützt). Die enzymatische Umsetzung des Chromogens Tetramethylbenzidindihydrochlorid wird durch Zugabe von 100 µl 0,5 N Schwefelsäure unterbrochen und die Extinktion bei 450 nm photometrisch bestimmt. Die gemessene Extinktion ist dabei der in der Probe enthaltenen Anti-HAV-Antikörper-Konzentration indirekt proportional

### e): Vergleich des erfindungsgemäßen Verfahrens mit einem alternativen, vollmonoklonalen Verfahren.

Das erfindungsgemäße Verfahren ist ein Enzymimmunotest nach dem 1-Schritt-Verfahren, das einen auf der Festphase gebundenen polyklonalen Anti-HAV-spezifischen Antikörper humanen Ursprungs und als Konjugat-Antikörper einen monoklonalen Anti-HAV-spezifischen Antikörper der Maus enthält. Das Vergleichsverfahren unterscheidet sich vom erfindungsgemäßen Verfahren darin, daß der polyklonale Festphasen-Antikörper durch einen monoklonalen Antikörper ersetzt wird.

In Tabelle 1 ist das erfindungsgemäße Verfahren mit dem alternativen, vollmonoklonalen Verfahren in Bezug auf Signalintensität der negativen Kontrolle und ermittelter Nachweisgrenze dargestellt.

**Tabelle 1:**

| **Vergleich des erfindungsgemäßen Verfahrens mit einem vollmonoklonalen Verfahren** | | |
|---|---|---|
| | Extinktionswert der negativen Kontrolle | Nachweisgrenze |
| Erfindungsgemäßes Verfahren | 1427 mE | 13 IU/I |
| | | |
| Vollmonoklonales Verfahren | 543 mE | 28 IU/I |

Die höhere Signalintensität der negativen Kontrolle und die verbesserte Nachweisgrenze des aus einem polyklonalen und einem monoklonalen. Anti-HAV-Antikörpers aufgebauten erfindungsgemäßen Verfahrens zeigen deutlich die Überlegenheit des erfindungsgemäßen Verfahrens gegenüber einem alternativen, vollmonoklonalen Verfahren.

### Beschreibung der Figuren

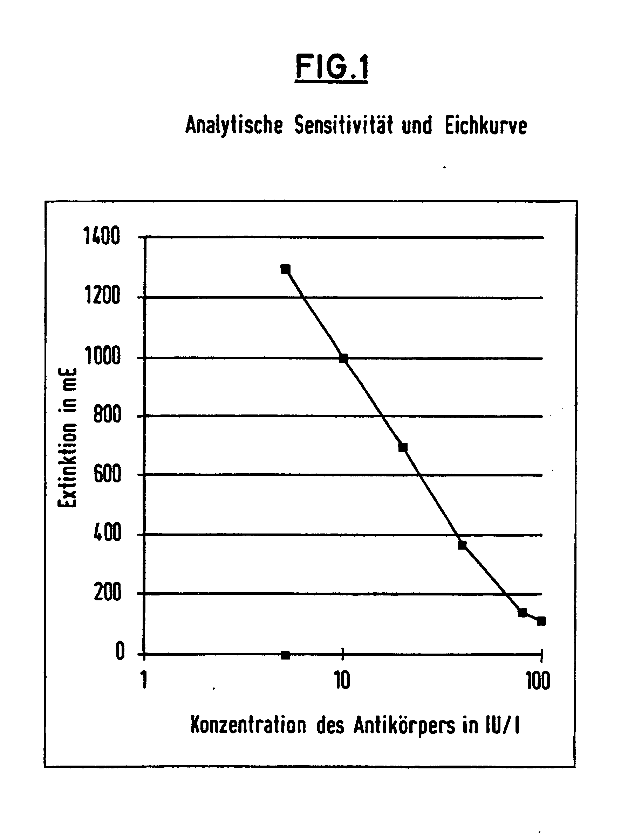

Die Nachweisgrenze des erfindungsgemäßen Verfahren wurde anhand von definierten Verdünnungen eines Anti-HAV-Standards (WHO-Standard) bestimmt. In Fig. 1 sind die gemessenen Extinktionswerte verschiedener Antikörperkonzentrationen in einer halblogarithmischen Darstellung abgebildet. Bedingt durch den kompetitiven Testaufbau nehmen die Extinktionswerte mit steigender Antikörperkonzentration ab.

Die berechnete analytische Sensitivität beträgt 13.4 IU/l, wobei als cut-off Wert der Extinktionswert der negativen Kontrolle halbiert wurde.

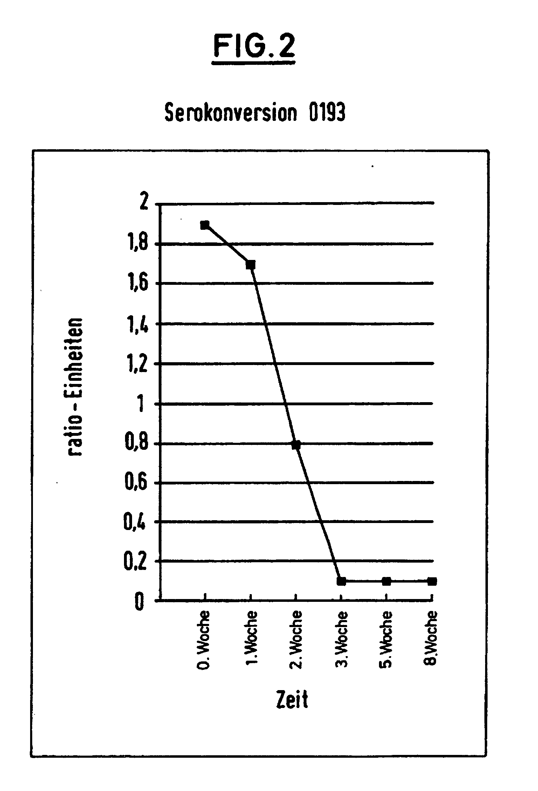

Anhand von Impfserokonversionen konnte gezeigt werden, daß niederaffine Antikörper gegen HAV mit dem erfindungsgemäßen Verfahren in einem sehr frühen Stadium nachweisbar sind. In Fig. 2 ist der zeitliche Verlauf einer typischen Anti-HAV-Serokonversion (0193) dargestellt. Serumproben wurden zu verschiedenen Zeiten vom Impf-Probanden abgenommen und diese mit dem erfindungsgemäßen Verfahren untersucht. Die dargestellten ratio-Werte entprechen dabei Quotientwerte aus den gemessenen Extinktionen und dem cut-off Wert. Ratio-Werte < 1 zeigen an, daß mit dem nachweisbaren Auftreten von Anti-HAV-Antikörpern eine Serokonversion stattgefunden hat. Wie in Fig. 2 dargestellt, konnten bereits ab der zweiten Woche nach erfolgter Impfung mit dem erfindungsgemäßen Verfahren eine Serokonversion nachgewiesen werden.

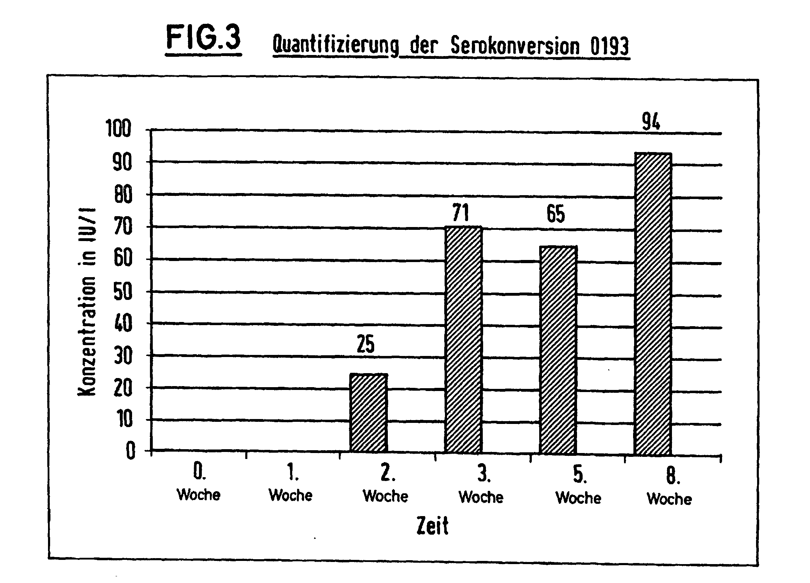

In Fig.3 wurden die gemessenen Extinktionswerte der untersuchten Serokonversion 0193 mit Hilfe der Eichkurve von Fig. 1 quantifiziert. Bereits ab der 2. Woche wurde eine Antikörperkonzentration bestimmt, die deutlich die Impfschutzgrenze von 20 IU/I überschreitet.

## Patentansprüche

1. Immunchemisches Verfahren zur Bestimmung eines Analyten mittels eines heterogenen kompetitiven Bestimmungsverfahrens, welches folgende Schritte einschließt:
a) Inkubation des Analyt mit einem ersten, einem zweiten und einem dritten spezifischen Bindungspartner, wobei der erste spezifische Bindungspartner an eine wasserunlösliche Festphase gebunden ist und der zweite spezifische Bindungspartner mit einer signalerzeugenden Markierung versehen ist, und der Analyt und der erste und der zweite spezifische Bindungspartner um die Bindung an den dritten spezifischen Bindungspartner konkurrieren,
b) Trennung der über den dritten spezifischen Bindungspartner an die Festphase gebundenen signalerzeugenden Markierung von dem ungebundenen Anteil,
c) Messung des durch den gebundenen Anteil der Markierung generierbaren Signals, und
d) Bestimmung der Analytkonzentration durch Vergleich der in Schritt c) gefundenen Werte mit einer unter gleichen Bedingungen aufgestellten oder theoretisch berechneten Standardkurve,
**dadurch gekennzeichnet, daß** der Analyt ein Hepatitis A-Virus- (HAV-) spezifischer Antikörper ist, daß als erster spezifischer Bindungspartner ein polyklonaler gegen HAV gerichteter Antikörper oder entsprechende Antikörperfragmente, als zweiter spezifischer Bindungspartner ein monoklonaler gegen HAV gerichteter Antikörper oder entsprechende Antikörperfragmente und als dritter spezifischer Bindungspartner ein HAV-Antigen verwendet wird, und daß der erste und zweite spezifische Bindungspartner unterschiedliche Bindungsstellen auf dem dritten Bindungspartner erkennen.

2. Verfahren nach Anspruch 1, **dadurch gekennzeichnet, daß** man als immunchemische Bestimmungsmethode das 1-Schritt-Verfahren anwendet.

3. Verfahren nach Anspruch 1, **dadurch gekennzeichnet, daß** man als signalerzeugende Komponente ein Enzym verwendet.

4. Verfahren nach Anspruch 3, **dadurch gekennzeichnet, daß** man als Enzym Meerrettich-Peroxidase verwendet.

5. Verfahren nach Anspruch 1, **dadurch gekennzeichnet, daß** der zu bestimmende Analyt ein Anti-HAV-spezifischer Antikörper der Subklassen IgM und/oder IgG ist.

6. Verfahren nach Anspruch 1, **dadurch gekennzeichnet, daß** der dritte spezifische Bindungspartner in einer niedrigen Reinigungsstufe eingesetzt wird, die einen spezifischen Proteingehalt von weniger als 80 % aufweist.

7. Verfahren nach Anspruch 6, **dadurch gekennzeichnet, daß** der dritte spezifische Bindungspartner in einer niedrigen Reinigungsstufe eingesetzt wird, die einen spezifischen Proteingehalt von weniger als 50 % aufweist.

8. Verfahren nach Anspruch 7, **dadurch gekennzeichnet, daß** der dritte spezifische Bindungspartner in einer niedrigen Reinigungsstufe eingesetzt wird, die einen spezifischen Proteingehalt von weniger als 20 % aufweist.

9. Verfahren nach Anspruch 1, **dadurch gekennzeichnet, daß** der dritte spezifische Bindungspartner ein in Zellkultur gezüchtetes HAV-Antigen ist.

## Claims

1. An immunochemical process for the determination of an analyte by means of a heterogeneous competitive determination process, which includes the following steps:
a) incubation of the analyte with a first, a second and a third specific binding component, the first specific binding component being bound to a water-insoluble solid phase and the second specific binding component being provided with a signal-generating label, and the analyte and the first and the second specific binding components competing for binding to the third specific binding component,
b) separation of the signal-generating label bound to the solid phase via the third specific binding component from the unbound fraction,
c) measurement of the signal which can be generated by the bound fraction of the label, and
d) determination of the analyte concentration by comparison of the values found in step c) with a standard curve plotted under identical conditions or calculated theoretically,
**characterized in that** the analyte is a hepatitis A virus (HAV) specific antibody, **in that** the first specific binding component used is a polyclonal anti-HAV antibody or corresponding antibody fragments, the second specific binding component used is a monoclonal anti-HAV antibody or corresponding antibody fragments and the third specific binding component used in an HAV antigen, and **in that** the first and second specific binding components recognize different binding sites on the third binding component.

2. The process as claimed in claim 1, **characterized in that** the immunochemical determination method used is the 1-step process.

3. The process as claimed in claim 1, **characterized in that** an enzyme is used as the signal-generating component.

4. The process as claimed in claim 3, **characterized in that** horseradish peroxidase is used as the enzyme.

5. The process as claimed in claim 1, **characterized in that** the analyte to be determined is an anti-HAV-specific antibody of the subclasses IgM and/or IgG.

6. The process as claimed in claim 1, **characterized in that** the third specific binding component is employed in a low purification state which has a specific protein content of less than 80%.

7. The process as claimed in claim 6, **characterized in that** the third specific binding component is employed in a low purification state which has a specific protein content of less than 50%.

8. The process as claimed in claim 7, **characterized in that** the third specific binding component is employed in a low purification state which has a specific protein content of less than 20%.

9. The process as claimed in claim 1, **characterized in that** the third specific binding component is an HAV antigen raised in cell culture.

## Revendications

1. Procédé immunochimique pour déterminer un analyte au moyen d'un procédé de détermination compétitif hétérogène, qui comprend les étapes suivantes :
a) incubation de l'analyte avec un premier, un deuxième et un troisième ligands spécifiques, le premier ligand spécifique étant lié à une phase solide insoluble dans l'eau et le deuxième ligand spécifique étant pourvu d'un marquage générant un signal, et l'analyte et les premier et deuxième ligands spécifiques concourant pour la liaison au troisième ligand spécifique,
b) séparation du marquage générant un signal lié à la phase solide, de portion non liée, au moyen du troisième ligand spécifique,
c) mesure du signal pouvant être généré par la portion liée du marquage, et
d) détermination de la concentration d'analytes par comparaison des valeurs trouvées à l'étape c) à l'aide d'une courbe de référence calculée de manière théorique ou établie dans les mêmes conditions,
**caractérisé en ce que** l'analyte est un anticorps spécifique du virus de l'hépatite A (VHA), **en ce qu'**on utilise en tant que premier ligand spécifique un anticorps polyclonal dirigé contre le VHA ou des fragments d'anticorps correspondants, en tant que deuxième ligand spécifique un anticorps monoclonal dirigé contre le VHA ou des fragments d'anticorps correspondants et en tant que troisième ligand spécifique un antigène VAH, et **en ce que** les premier et deuxième ligands spécifiques reconnaissent des sites de liaison différents sur le troisième ligand.

2. Procédé selon la revendication 1, **caractérisé en ce qu'**on applique le procédé à 1 étape en tant que méthode de détermination immunochimique.

3. Procédé selon la revendication 1, **caractérisé en ce qu'**on utilise un enzyme en tant que composé générant un signal.

4. Procédé selon la revendication 3, **caractérisé en ce qu'**on utilise en tant qu'enzyme de la peroxydase de raifort.

5. Procédé selon la revendication 1, **caractérisé en ce que** l'analyte à déterminer est un anticorps spécifique anti-VAH des sous-classes IgM et/ ou IgG.

6. Procédé selon la revendication 1, **caractérisé en ce que** le troisième ligand spécifique est utilisé dans une étape de lavage inférieure, laquelle présente une teneur en protéine spécifique de moins de 80 %.

7. Procédé selon la revendication 6, **caractérisé en ce que** le troisième ligand spécifique est utilisé dans une étape de lavage inférieure, laquelle présente une teneur en protéine spécifique de moins de 50 %.

8. Procédé selon la revendication 7, **caractérisé en ce que** le troisième ligand spécifique est utilisé dans une étape de lavage inférieure, laquelle présente une teneur en protéine spécifique de moins de 20 %.

9. Procédé selon la revendication 1, **caractérisé en ce que** le troisième ligand spécifique est un antigène VAH produit dans une culture cellulaire.
